(19) Europäisches Patentamt
European Patent Office
Office européen des brevets

(11) Numéro de publication : **0 087 375**
**B1**

(12) **FASCICULE DE BREVET EUROPÉEN**

(45) Date de publication du fascicule du brevet : **18.09.85**

(51) Int. Cl.[4] : **C 07 D235/24**, A 61 K 31/415

(21) Numéro de dépôt : **83420012.3**

(22) Date de dépôt : **25.01.83**

(54) **Nouveaux dérivés du cyano-2 benzimidazole, leur préparation et leur utilisation comme fongicides et acaricides.**

(30) Priorité : **09.02.82 FR 8202281**

(43) Date de publication de la demande :
**31.08.83 Bulletin 83/35**

(45) Mention de la délivrance du brevet :
**18.09.85 Bulletin 85/38**

(84) Etats contractants désignés :
**AT BE CH DE FR GB IT LI LU NL SE**

(56) Documents cités :
**FR-A- 2 051 493**
**FR-A- 2 070 090**
**Le dossier contient des informations techniques présentées postérieurement au dépôt de la demande et ne figurant pas dans le présent fascicule.**

(73) Titulaire : **RHONE-POULENC AGROCHIMIE**
**14-20, rue Pierre Baizet**
**F-69009 Lyon (FR)**

(72) Inventeur : **Giraudon, Raymond**
**5 Rue des Colverts**
**F-77330 Lesigny (FR)**
Inventeur : **Santini, Georges**
**7 Rue Jeanne d'Arc**
**F-94320 Thiais (FR)**

(74) Mandataire : **Chaumette, Michel et al**
**RHONE-POULENC AGROCHIMIE BP 9163-09**
**F-69263 Lyon Cedex 1 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 087 375 B1

**Description**

La présente demande de brevet concerne de nouveaux dérivés du cyano-2 benzimidazole ainsi que la préparation de ces composés. Elle concerne de plus les compositions pesticides, notamment antifongiques et acaricides utilisables en agriculture et contenant comme matière active un de ces composés ainsi que les traitements pesticides et plus particulièrement les traitements antifongiques et acaricides effectués au moyen de ces composés.

Le brevet américain n° 3 576 818 décrit des dérivés du cyano-2 benzimidazole répondant à la formule (I)

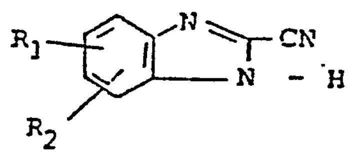

(I)

dans laquelle $R_1$ et $R_2$ sont choisis dans le groupe comprenant l'hydrogène, le chlore, le brome, $NO_2$ et les radicaux alkyle et alkoxy contenant au plus 4 atomes de carbone. Ce brevet américain indique que ces composés (I) sont utilisables comme bactéricides et agents antiseptiques, il décrit dans son exemple n° 1 le cyano-2 benzimidazole, c'est-à-dire le composé selon la formule (I) pour lequel $R_1$ et $R_2$ représentent l'atome d'hydrogène.

La demande de brevet français n° 2 051 493 décrit des dérivés du cyano-2 benzimidazole pour lesquels l'atome d'azote en position 1 sur le cycle benzimidazole peut-être substitué par un atome d'hydrogène ou un radical alkyle ou alkoxycarbonyle. Elle signale que ces composés ont une activité biocide et sont utilisables comme fongicides, anthelmintiques, insecticides et herbicides. L'exemple n° 4 de cette demande de brevet français décrit le cyano-2 benzimidazole, déjà mentionné ci-dessus.

La demande de brevet français n° 2 070 090 décrit des dérivés du cyano-2 benzimidazole pour lesquels l'atome d'azote en position 1 sur le cycle benzimidazole peut-être substitué par l'atome d'hydrogène ou un radical alkyle, alkoxyalkyle alcényle, carboxyalkyle, alcanoyle, aminoalkyle, hydroxy, alkoxy, carboxyalkoxy, sulfoalkoxy et aminoalkoxy et enseigne que ces composés sont utilisables comme agents anthelmintiques, comme agents anti-coccidies et comme pesticides par exemple herbicides et bactéricides.

La présente demande de brevet concerne des dérivés du cyano-2 benzimidazole différents de ceux décrits dans les documents cités plus haut et présentant de remarquables propriétés antifongiques. Certains de ces nouveaux composés présentent de plus d'intéressantes propriétés acaricides.

Les nouveaux dérivés du cyano-2 benzimidazole selon l'invention sont caractérisés en ce qu'ils répondent à la formule générale : (II)

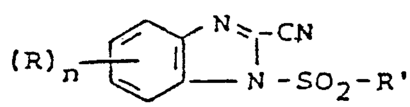

(II)

dans laquelle n, R et R' ont les significations indiquées ci-après, étant entendu que, dans ce qui suit, sauf indication contraire, l'adjectif « inférieur » appliqué à un radical organique signifie que ce radical comporte au plus six atomes de carbone.

Dans la formule (II) :

n représente un nombre entier pouvant être égal à 0, 1, 2 ou 3.

R représente un atome d'halogène (de préférence un atome de chlore ou de fluor ou de brome) ou un radical alkyle inférieur éventuellement substitué par un ou plusieurs atomes d'halogènes (tel que, par exemple, le radical méthyle ou le radical trifluorométhyle) ; alkoxy inférieur éventuellement substitué par un ou plusieurs atomes d'halogènes ou par le radical phényle (tel que, par exemple, le radical méthoxy ou le radical trifluorométhoxy ou le radical benzyloxyl) : alkylthio inférieur éventuellement substitué par un ou plusieurs atomes d'halogènes ; alcényle inférieur ; alcynyle inférieur ; amino ; nitro ; cyano ; cyanato (CNO-) ; thiocyanato (NCS-) ; isothiocyanato (SCN-) ; alkylsulfonyle inférieur ; sulfamoyle alkylsulfinyle inférieur benzoyle ; alkoxycarbonyle comportant de 2 à 5 atomes de carbone (par exemple méthoxycarbonyle), étant entendu que, lorsque n est supérieur ou égal à 2, les substituants R peuvent être soit identiques, soit différents,

R' représente un radical alkyle ou cycloalkyle/inférieurs éventuellement substitués par un ou plusieurs atomes d'halogènes (tel que, par exemple, les radicaux méthyle, éthyle, isopropyle, trichlorométhyle etc...) ; ou un radical amino, éventuellement substitué par un ou deux radicaux alkyles inférieurs, identiques ou différents, eux-mêmes par un alkoxy inférieur par deux radicaux formant avec cet atome d'azote un hétérocycle, comportant de 4 à 6 chaînons et de 1 à 3 hétéroatomes dans le cycle (tel que par exemple le radical morpholino, le radical pyrrolidino etc...).

Au sens du présent texte, on entendra que les radicaux ou parties alkyles peuvent être soit linéaires, soit ramifiés. Il en est de même des radicaux alcényles et alcynyles.

Parmi les composés répondant à la formule générale (II), une sous-famille préférée en raison de ses remarquables propriétés antifongiques est constituée par les composés selon la formule (II) pour lesquels :

n et R ont la même signification que précédemment,

R' représente le radical diméthylamino, ou un radical alkyle comportant de 1 à 3 atomes de carbone éventuellement substitué par un ou plusieurs halogènes, ou un atome d'azote substitué par deux radicaux formant avec cet atome d'azote un hétérocycle comportant de 4 à 6 chainons et comportant éventuellement comme deuxième hétéroatome dans le cycle (c'est-à-dire en plus de l'atome d'azote) l'atome d'oxygène.

Parmi ces composés antifongiques préférés, une sous-famille préférée est constituée par les composés répondant à la formule (II), pour lesquels :

n a la même signification que précédemment,

R représente un atome d'halogène ou un radical alkyle comportant de 1 à 3 atomes de carbone ; trifluorométhyle ; alkoxy éventuellement halogéné comportant de 1 à 3 atomes de carbone ; amino ; nitro ; cyano ; alkylsulfonyle comportant de 1 à 3 atomes de carbone, les substituants R pouvant être soit identiques soit différents,

R' représente le radical diméthylamino ou un radical alkyle comportant de 1 à 3 atomes de carbone, (avantageusement le radical isopropyle) ou le radical pyrrolidino, ou le radical morpholino.

Parmi les composés répondant à la formule générale (II) il a été observé que ceux pour lesquels n est égal à un, deux ou trois et l'un au moins des substituants R représente le radical trifluorométhyle possédaient, en plus de leurs propriétés antifongiques, une activité acaricide intéressante, notamment vis-à-vis des acariens phytophages.

L'invention concerne de plus un procédé pour préparer les composés selon la formule (II).

Ce procédé est caractérisé en ce qu'il consiste à faire réagir un cyano-2 benzimidazole de formule (III)

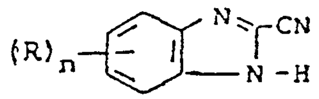

(III)

dans laquelle R et n ont la même signification que dans la formuel (II), ou un sel de métal alcalin ou d'ammonium de ce cyano-2 benzimidazole (III), sur un halogénure de formule (IV)

$$X—SO_2R' \qquad (IV)$$

dans laquelle R' a la même signification que dans la formule (II) et X représente un atome d'halogène, de préférence un atome de chlore.

La réaction du cyano-2 benzimidazole (III) sur l'halogénure (IV) s'effectue avantageusement en présence d'un accepteur d'acide, en milieu anhydre ou non anhydre, dans un solvant, inerte dans les conditions de la réaction, généralement à la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine. Comme solvants on utilise avantageusement des solvants aprotiques polaires, tels que par exemple, le diméthylformamide, le diméthylacétamide, le diméthylsulfoxyde, l'acétone, la méthyl-éthyl-cétone, l'acétonitrile, la N-méthylpyrrolidone. Si désiré, cette réaction peut être effectuée en présence d'un catalyseur approprié. Comme catalyseur utilisable, on peut mentionner des catalyseurs de transfert de phases, tels que, par exemple, des dérivés d'ammonium quaternaire.

La réaction du sel de métal alcalin ou d'ammonium du cyano-2 benzimidazole (III) sur l'halogénure (IV) ne nécessite pas la présence d'un accepteur d'acide. Elle s'effectue en milieu anhydre ou non anhydre, dans un solvant, inerte dans les conditions de la réaction, généralement à la température d'ébullition du solvant. Les solvants aprotiques polaires cités plus haut peuvent être utilisés avantageusement dans cette réaction.

Si désiré, cette réaction peut être effectuée en présence d'un catalyseur approprié, tel que, par exemple, un catalyseur de transfert de phases (par exemple un dérivé d'ammonium quaternaire).

Le sel de métal alcalin ou d'ammonium du composé (III) est préparé dans une opération préalable, éventuellement effectuée in situ, par action d'une base appropriée (par exemple soude, potasse, ammoniaque) ou d'un carbonate de métal alcalin, ou d'un alcoolate de métal alcalin (par exemple méthylate de sodium ou éthylate de sodium ou potassium) sur ce composé (III).

En fin de réaction, quelque soit le procédé utilisé, le composé formé est isolé du milieu réactionnel par tout moyen en soi connu tel que, par exemple par distillation du solvant, ou par cristallisation du produit dans le milieu réactionnel, our par filtration, puis, si nécessaire, ce composé est purifié par des méthodes usuelles telles que recristallisation dans un solvant approprié.

Le cyano-2 benzimidazole de formule (III), servant de produit de départ peut être préparé selon l'une ou l'autre des méthodes A, B et C décrites ci-après :

Méthode A :

Action de l'ammoniaque sur un trihalogénométhyl-2 benzimidazole, selon le procédé décrit dans le brevet américain n° 3 576 818 cité plus haut,

Méthode B :

Action du trichlorure de phosphore sur un cyano-2 hydroxy-1 benzimidazole de formule (V)

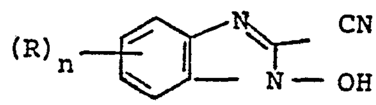

(V)

dans laquelle R et n ont la même signification que dans la formule (II), en opérant dans l'acétone chauffée à la température du reflux.

Le cyano-2 hydroxy-1 benzimidazole (V) peut être obtenu à partir du (nitro-2 phényl) amino-2 acétonitrile de formule (VI)

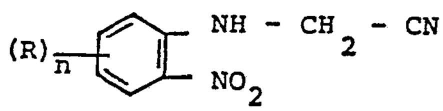

(VI)

dans laquelle R et n ont la même signification que précédemment, par chauffage en présence de carbonate de potassium, selon la méthode décrite par L. KONOPSKI et B. SERAFIN dans Bocz. Chem. *51* 1783 (1977).

Le composé (VI) peut être obtenu à partir de la nitro-2 aniline correspondante en opérant selon la méthode décrite par K. DINROTH et H.C. AURICH dans Chem. Ber. *98* 3902 (1965).

Méthode C :

Action d'un agent de déshydratation tel que $POCl_3$ ou $SOCl_2$ sur l'hydroxyiminométhyl-2 benzimidazole de formule (VII)

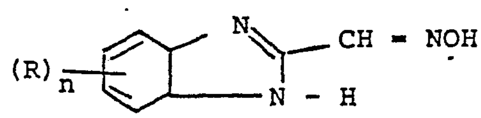

(VII)

dans laquelle R et n ont la même signification que dans la formule (II)

Les exemples ci-après, décrits à titre non limitatif, illustrent la préparation des composés selon l'invention et leur utilisation comme fongicides et acaricides. Les structures des composés décrits dans ces exemples ont été confirmées par spectrométrie de résonance magnétique nucléaire (RMN) et/ou par spectrométrie infrarouge.

Exemple 1 : Préparation du diméthylsulfanoyl-1 cyano-2 benzimidazole (composé 1) de formule :

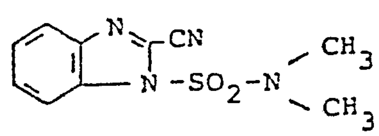

(Composé 1)

A une solution de 10 g de cyano-2 benzimidazole dans 100 ml d'acétonitrile, on ajoute 4,83 g de carbonate de potassium anhydre, puis 7ml de chlorure de N,N-diméthyl-sulfamoyle et 1,18 g de hydrogénosulfate de tétrabutyl-ammonium.

Le mélange est alors chauffé à reflux pendant 3 heures. Après refroidissement à 25 °C environ, on ajoute 300 ml d'acétate d'éthyle. Le mélange réactionnel est ensuite filtré et le filtrat concentré sous pression réduite (0,032 bar). Le solide obtenu est recristallisé dans 200 ml d'éther isopropylique.

On obtient ainsi 5,3 g (rendement 30,3 %) de diméthylsulfamoyl-1 cyano-2 benzimidazole fondant à 93 °C :

Composition élémentaire

| | | | | | |
|---|---|---|---|---|---|
| % calculé | C 47,99 | H 4,03 | N 22,38 | O 12,78 | S 12,81 |
| % trouvé | C 48,31 | H 4,10 | N 22,46 | O 12,27 | S 13,00 . |

Le cyano-2 benzimidazole utilisé comme produit de départ a été obtenu par action de l'ammoniaque sur le trichlorométhyl-2 benzimidazole, selon la méthode décrite dans l'exemple n° 1 du brevet américain n° 3 576 818.

Le cyano-2 benzimidazole a également été préparé comme indiqué ci-après :

En une heure on ajoute 79 g d'hydroxyiminométhyl-2 benzimidazole à 300 ml de chlorure de sulfonyle ; l'addition est exothermique et provoque un dégagement gazeux. Le mélange réactionnel est encore chauffé 2 heures 30 au reflux puis, après refroidissement, dilué par 200 ml d'éther de pétrole. Le précipité obtenu est filtré puis lavé par une solution de carbonate de potassium.

Après recristallisation dans un mélange acétonitrile/acétate d'éthyle on obtient 42,5 g (60,6 %) de cyano-2 benzimidazole fondant à 268 °.

Exemple 2 : Préparation du diméthylsulfamoyl-1 cyano-2 chloro-5 benzimidazole (composé 2A) et du diméthyl-sulfamoyl-1 cyano-2 chloro-6 benzimidazole (composé 2B)

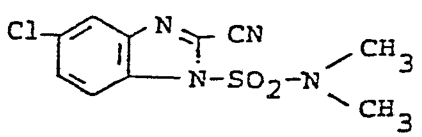

Composé 2A

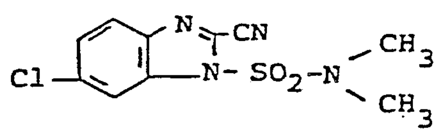

Composé 2B

A une suspension de 8 g de cyano-2 chloro-5 benzimidazole dans 100 ml d'acétone séchée sur $K_2CO_3$, on ajoute 2,87 g de potasse en écailles.

Après 30 minutes environ, la potasse est dissoute et le mélange réactionnel est devenu presque homogène.

On ajoute alors en une fois 4,85 ml de chlorure de diméthylsulfamoyle et le mélange réactionnel est agité pendant 18 heures. Ce mélange réactionnel est ensuite filtré puis le filtrat est concentré sous pression réduite (0,026 bar) à 50 °C environ. On obtient ainsi un solide blanc rosé que l'on recristallise dans 150 ml d'isopropanol et on obtient alors 6,5 g d'un solide blanc fondant à 130 °C.

Par spectrographie RMN et Infra-rouge, ce composé est analysé comme un mélange, en proportions sensiblement égales, de diméthylsulfamoyl-1 cyano-2 chloro-5 benzimidazole, et de diméthylsulfamoyl-1 cyano-2 chloro-6 benzimidazole, ce mélange d'isomères ayant la composition élémentaire ci-après :

| | | | | | | |
|---|---|---|---|---|---|---|
| % calculé | C 42,18 | H 3,18 | C 12,45 | N 19,68 | O 11,24 | S 11,26 |
| % trouvé | C 42,28 | H 3,18 | C 12,41 | N 19,42 | O 11,27 | S 11,20 |

Ces deux isomères sont ensuite séparés par chromatographie sur silice sous légère pression (chromatographie flash) et on obtient ainsi séparés le diméthylsulfamoyl-1 cyano-2 chloro-5 benzimidazole (composé 2A) et le diméthylsulfamoyl-1 cyano-2 chloro-6 benzimidazole (composé 2B). L'un de ces composés fond à 147 °C et l'autre à 167 °C.

Exemple 3 : Préparation de l'isopropylsulfonyl-1 cyano-2 trifluorométhyl-5 benzimidazole (composé 3A) et de l'isopropylsulfonyl-1 cyano-2 trifluorométhyl-6 benzimidazole (composé 3B)

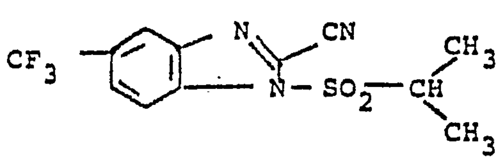

Composé 3A

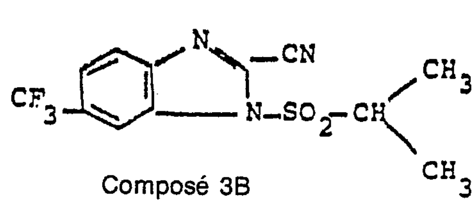

Composé 3B

A une solution de 12 g de cyano-2 trifluorométhyl-5 benzimidazole dans 100 ml d'acétone, on ajoute 3,62 g de potasse en écailles.

Après 15 minutes d'agitation à 25 °C, on ajoute 6,35 ml de chlorure d'isopropylsulfonyle. Le mélange est ensuite maintenu pendant deux heures à la température de reflux du solvant, puis concentré après refroidissement. Le résidu solide obtenu est dissous dans 250 ml de chlorure de méthylène et la solution obtenue est lavée par deux fois 200 ml d'eau distillée. Après séchage, la solution dans le chlorure de

méthylène est concentrée, puis le résidu obtenu est recristallisé dans 50 ml d'isopropanol. On obtient ainsi 11,2 g (rendement 61,9 %) d'un solide blanc fondant à 120 °C.

Par spectrographie RMN et infra-rouge, ce composé est analysé comme étant un mélange, en proportions sensiblement égales de l'isopropylsulfonyl-1 cyano-2 trifluorométhyl-5 benzimidazole (composé 3A) et de l'isopropylsulfonyl-1 cyano-2 trifluorométhyl-6 benzimidazole (composé 3B), ce mélange d'isomères ayant la composition élémentaire ci-après :

% calculé C 45,42 H 3,18 F 17,96 N 13,24 S 10,10
% trouvé C 45,56 H 3,03 F 17,93 N 13,07 S 10,07

Exemple n° 4 : Préparation du méthylsulfonyl-1 cyano-2 trifluorométhyl-5 benzimidazole (composé 4A) et du méthylsulfonyl-1 cyano-2 trifluorométhyl-6 benzimidazole (composé 4B)

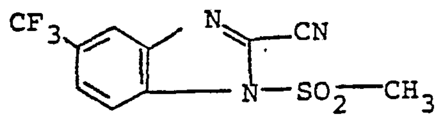

(Composé 4A)

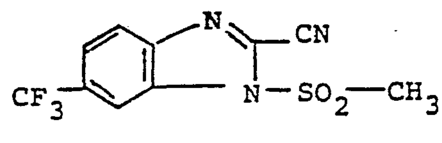

(Composé 4B)

On opère selon la méthode décrite dans l'exemple n° 3 à partir des matières premières appropriées, si ce n'est que le solvant de recristallisation est le toluène (au lieu de l'isopropanol).

On obtient ainsi un mélange (78/22) des composés 4A et 4B.

Point de fusion du mélange : 142 °C

Composition élémentaire du mélange

% calculé C 41,52 N 2,09 N 14,53 S 11,08
% trouvé C 42,85 N 2,08 N 15,22 S 10,10

Exemple 5 : Préparation du diméthylsulfamoyl-1 cyano-2 trifluorométhyl-5 benzimidazole (composé 5A) et du diméthylsulfamoy-1 cyano-2 trifluorométhyl-6 benzimidazole (composé 5B)

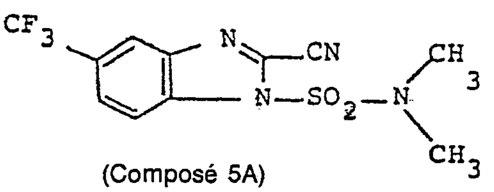

(Composé 5A)

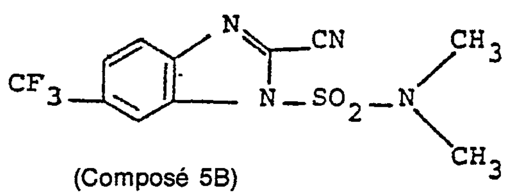

(Composé 5B)

On opère selon la méthode décrite dans l'exemple 3 à partir des matières premières appropriées.

On obtient ainsi un mélange, en proportions sensiblement égales des composés 5 A et 5 B

Point de fusion du mélange : 120 °C

Composition élémentaire du mélange :

% calculé C 41,51 H 2,85 F 17,91 N 12,60 O 10,05 S 10,07
% trouvé C 41,53 H 2,75 F 17,87 N 12,12 O 11,50 S 10,14

Exemple 6

En opérant selon l'une ou l'autre des méthodes décrites dans les exemples 1 et 3, à partir des matières premières appropriées, les composés n° 6A à 71B ont été préparés. Les formules et points de fusion de ces composés ou de leurs mélanges ainsi que des composés ou mélanges de composés décrits dans les exemples précédents sont reportés dans le tableau ci-après.

Dans ce tableau :

— dans la colonne R, le chiffre indiqué entre parenthèses avant le substituant indique la position de ce substituant sur le noyau benzimidazole,

— dans la colonne R', l'indication « idem » signifie que pour le composé considéré, le radical R' a même signification que pour le composé immédiatement antérieur dans ce tableau. Cette notation a été utilisée systématiquement dans le cas où on obtient un mélange d'isomères (A et B) qui ne diffèrent entre eux que par la position du ou des substituants R sur le cycle benzimidazole.

— dans la colonne « point de fusion », l'indication « M » signifie que le point de fusion indiqué est celui d'un mélange comprenant les deux composés dans les proportions pondérales indiquées après la lettre M.

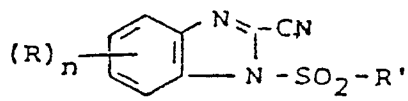

| N° | n | R | R' | Point de fusion |
|---|---|---|---|---|
| 1 | 0 | | $-N(CH_3)_2$ | 93°C |
| 2A<br>2B | 1<br>1 | (5)-Cl<br>(6)-Cl | $-N(CH_3)_2$<br>idem | M 50/50<br>: 130°C |
| 2A | 1 | (5)-Cl | $-N(CH_3)_2$ | 147°C |
| 2B | 1 | (6)-Cl | idem | 167°C |
| 3A<br>3B | 1<br>1 | $(5)-CF_3$<br>$(6)-CF_3$ | $-CH(CH_3)_2$<br>idem | M 50/50<br>: 120°C |
| 4A<br>4B | 1<br>1 | $(5)-CF_3$<br>$(6)-CF_3$ | $CH_3$<br>idem | M 78/22<br>: 142°C |
| 5A<br>5B | 1<br>1 | $(5)-CF_3$<br>$(6)-CF_3$ | $-N(CH_3)_2$<br>$-N(CH_3)_2$ | M 50/50<br>: 120°C |
| 6A<br>6B | 1<br>1 | $(5)-CH_3$<br>$(6)-CH_3$ | $-N(CH_3)_2$<br>idem | M 50/50<br>: 110°C |
| 7A<br>7B | 1<br>1 | $(5)-NO_2$<br>$(6)-NO_2$ | $-N(CH_3)_2$<br>idem | M 50/50<br>: 178°C |
| 7A | 1 | $(5)-NO_2$ | $-N(CH_3)_2$ | 207°C |
| 7B | 1 | $(6)-NO_2$ | $-N(CH_3)_2$ | 190°C |
| 8 | 2 | $(5)-CH_3,(6)-CH_3$ | $-N(CH_3)_2$ | 149°C |
| 9 | 2 | (4)-Cl,(6)-Cl | $-N(CH_3)_2$ | 177°C |
| 10 | 1 | (4)-Cl | $-N(CH_3)_2$ | 130°C |
| 11 | 1 | $(5)-C(CH_3)_3$ | $-N(CH_3)_2$ | 125°C |

| N° | n | R | R' | Point de fusion |
|---|---|---|---|---|
| 12A<br>12B | 1<br>1 | (5)-CO-$C_6H_5$<br>(6)-CO-$C_6H_5$ | -$N(CH_3)_2$<br>idem | M 50/50<br>: 132°C |
| 13A<br>13B | 1<br>1 | (5)-$OCH_3$<br>(6)-$OCH_3$ | -$N(CH_3)_2$<br>-$N(CH_3)_2$ | M 50/50<br>: 125°C |
| 14 | 2 | (5)-Cl,(6)-Cl | -$N(CH_3)_2$ | 174°C |
| 15A<br>15B | 1<br>1 | (5)-F<br>(6)-F | -$N(CH_3)_2$<br>idem | M 50/50<br>109-110°C |
| 16 | 0 | | -$CH_3$ | 198°C |
| 17 | 2 | (4)-Cl,(6)-Cl | -$CH_3$ | 204°C |
| 18 | 1 | (4)-$CH_3$ | -$CH_3$ | 171°C |
| 19A<br>19B | 2<br>2 | (5)-Cl,(6)-$CH_3$<br>(5)-$CH_3$,(6)Cl | -$N(CH_3)_2$<br>idem | M 43/57<br>: 181°C |
| 20 | 1 | (4)-Cl | -$CH_3$ | 173°C |
| 21 | 1 | (4)-Cl | -$CH(CH_3)_2$ | 120°C |
| 22 | 0 | | -$CH(CH_3)_2$ | 118°C |
| 23 | 2 | (4)-Cl,(6)-Cl | -$CH(CH_3)_2$ | 176°C |
| 24 | 2 | (5)-Cl,(6)-Cl | -$CH(CH_3)_2$ | 181°C |
| 25A<br>25B | 1<br>1 | (5)-Br<br>(6)-Br | -$N(CH_3)_2$<br>idem | M 50/50<br>138-142°C |
| 26 | 1 | (4)-Br | -$N(CH_3)_2$ | 143°C |
| 27 | 2 | (4)-$NO_2$,(5)-$OCH_3$ | -$N(CH_3)_2$ | 185°C |
| 28 | 3 | (4)-$NO_2$,(5)-$OCH_3$<br>(6)-$OCH_3$ | -$N(CH_3)_2$ | 180°C |

| N° | n | F | R' | Point de fusion |
|---|---|---|---|---|
| 29A | 1 | (5)-CN | $-N(CH_3)_2$ | M 43/57 |
| 29B | 1 | (6)-CN | idem | 160°C à 220°C |
| 30A | 1 | (5)-Cl | 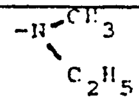 | M 55/45 : 91°C |
| 30B | 1 | (6)-Cl | idem | |
| 31A | 1 | $(5)-NO_2$ | $-CH(CH_3)_2$ | M 64/36 |
| 31B | 1 | $(6)-NO_2$ | idem | : 150°C |
| 32A | 1 | (5)-Cl | $-CH(CH_3)_2$ | M 30/70 |
| 32B | 1 | (6)-Cl | idem | : 120°C |
| 33A | 1 | $(5)-O-CH_2-C_6H_5$ | $-CH(CH_3)_2$ | M(40/60 ou 60/40 |
| 33B | 1 | $(6)-O-CH_2-C_6H_5$ | idem | : 135°C |
| 34A | 1 | $(5)-O-CH_2-C_6H_5$ | $-N(CH_3)_2$ | M(50/50) |
| 34B | 1 | $(6)-O-CH_2-C_6H_5$ | idem | : 120°C |
| 35A | 1 | (5)-F | 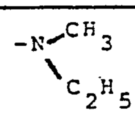 | M 50/50 : 96°C |
| 35B | 1 | (6)-F | idem | |
| 36A | 1 | $(5)-SCH_3$ | $-N(CH_3)_2$ | M 50/50 |
| 36B | 1 | $(6)-SCH_3$ | idem | : 108°C |
| 37A | 2 | $(5)-Cl,(6)-OCH_3$ | $-N(CH_3)_2$ | M 50/50 |
| 37B | 2 | $(5)-OCH_3,(6)-Cl$ | idem | 190°C et 205°C |
| 38 | 1 | $(5)-ou(6)-NH_2$ | $-N(CH_3)_2$ | 212°C |
| 39 | 2 | (4)-Br,(6)-Br | $-N(CH_3)_2$ | 215°C |
| 40A | 2 | $(5)-NO_2,(6)-Cl$ | $-N(CH_3)_2$ | M 50/50 |
| 40B | 2 | $(5)-Cl,(6)-NO_2$ | idem | : 184°C |
| 41A | 2 | $(5)-Cl,(6)-SO_2CH_3$ | $-N(CH_3)_2$ | M 50/50 |
| 41B | 2 | $(5)-SO_2CH_3,(6)-Cl$ | idem | : 191°C |

| N° | n | R | R' | Point de fusion |
|---|---|---|---|---|
| 42 | 1 | (4)-$NH_2$ | $-N(CH_3)_2$ | 140-150°C |
| 43 | 2 | (4)-$CH_3$,(6)-Br | $-N(CH_3)_2$ | 207°C |
| 44 | 1 | (5)-ou(6)-NCS | $-N(CH_3)_2$ | 118-120°C |
| 45 | 2 | (4)-Cl,(5)-Cl | $-N(CH_3)_2$ | 194-195°C |
| 46A<br>46B | 1<br>1 | (5)-$SO_2$-$NH_2$<br>(6)-$SO_2$-$NH_2$ | $-N(CH_3)_2$<br>idem | M 50/50<br>: 158°C |
| 47 | 1 | (6)-NCS | $-N(CH_3)_2$ | 115-120°C |
| 48 | 3 | (4)-Cl,(5)$OCH_3$<br>(6)-Cl | $-N(CH_3)_2$ | 160°C |
| 49 | 1 | (4)-$NO_2$ | $-N(CH_3)_2$ | 196°C |
| 50 | 2 | (4)-$CH_3$,(5)-Cl | $-N(CH_3)_2$ | 154°C |
| 51 | 3 | (3)-Cl,(4)-Cl,<br>(5)-Cl | $-N(CH_3)_2$ | 208-210°C |
| 52A<br>52B | 1<br>1 | (5)-SO-$CH_3$<br>(6)-SO-$CH_3$ | $-N(CH_3)_2$<br>idem | M 63/37<br>: 116°C |
| 53A<br>53B | 1<br>1 | (5)-$SO_2$-$CH_3$<br>(6)-$SO_2$-$CH_3$ | $-N(CH_3)_2$<br>$-N(CH_3)_2$ | M 55/45<br>: 166°C |
| 54A<br>54B | 1<br>1 | (5)-$CF_3$<br>(6)-$CF_3$ | $-CCl_3$<br>idem | M 65/35 :<br>114-116°C |
| 55 | 2 | (4)-$CH_3$,(6)-Cl | $-N(CH_3)_2$ | 185°C |
| 56A<br>56B | 2<br>2 | (5)-Cl,(6)-$CF_3$<br>(5)-$CF_3$,(6)-Cl | $-N(CH_3)_2$<br>idem | M 50/50<br>: 158°C |
| 57 | 2 | (4)-Cl,(6)-Cl | 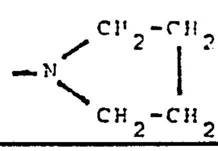 | 180°C |

| N° | n | F | P' | |
|---|---|---|---|---|
| 58A | 1 | (5)-$CF_3$ | 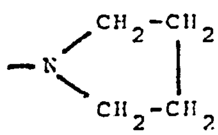 | M 50/50 125 à 130°C |
| 58B | 1 | (6)-$CF_3$ | idem | |
| 59A | 1 | (5)-$CF_3$ | 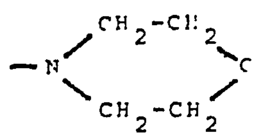 | M 50/50 155 à 165°C |
| 59B | 1 | (6)-$CF_3$ | idem | |
| 60A | 2 | (5)-Cl,(6)-$CF_3$ | -$CH(CH_3)_2$ | M 66/34 |
| 60B | 2 | (5)-$CF_3$,(6)-Cl | idem | : 161°C |
| 61 | 2 | (4)-$CF_3$,(6)-Cl | -$N(CH_3)$ | 188°C |
| 62A | 1 | (5)-CO-O-$CH_3$ | -$N(CH_3)_2$ | M 40/60 |
| 62B | 1 | (6)-CO-O-$CH_3$ | idem | : 132°C |
| 63A | 2 | (4)-Br,(5)-Cl | -$N(CH_3)_2$ | M 50/50 |
| 63B | 2 | (4)-Cl,(5)-Br | idem | 168-170°C |
| 64 | 2 | (4)-$CH_3$,(5)-$NO_2$ | $N(CH_3)_2$ | 194°C |
| 65A | 2 | (5)-$NO_2$,(6)-$CF_3$ | $N(CH_3)_2$ | M 60/40 |
| 65B | 2 | (5)-$CF_3$,(6)-$NO_2$ | idem | : 171°C |
| 66 | 2 | (4)-Cl,(6)-Cl | -$N(C_2H_5)_2$ | 153°C |
| 67 | 2 | (4)-$NO_2$,(5)-$CH_3$ | -$N(CH_3)_2$ | 173°C |
| 68A | 2 | (5)-$NO_2$,(6)-$CH_3$ | -$N(CH_3)_2$ | M(65/35 ou 35/65) |
| 68B | 2 | (5)-$CH_3$,(6)-$NO_2$ | idem | : 155°C |
| 69 | 2 | (4)-Cl,(6)-F | -$N(CH_3)_2$ | 151°C |
| 70 | 2 | (4)-$CF_3$,(6)-$CF_3$ | -$N(CH_3)_2$ | 168°C |
| 71A | 1 | (5)-$SCF_3$ | -$N(CH_3)_2$ | M (80/20) 131°C |
| 71B | 1 | (6)-$SCF_3$ | -$N(CH_3)_2$ | |

Exemple 7 : Tests en serre sur mildiou de la tomate

Des plants de tomate (Lycopersicum esculentum), de variété Marmande, sont cultivés dans des godets. Lorsque ces plants sont âgés d'un mois (stade 5 à 6 feuilles, hauteur 12 à 15 cm), ils sont traités par pulvérisation au moyen d'une suspension ou solution aqueuse de la matière à tester, à la concentration désirée et contenant 0,02 % d'un condensat de monoléate de sorbitan et de 20 molécules d'oxyde d'éthylène. Chaque plant de tomate reçoit environ 5 ml de la solution ou dispersion. Pour chaque concentration de matière active à tester, le traitement est effectué sur huit plants. Des plants utilisés comme témoins sont traités par une solution ne contenant pas de matière active, mais contenant 0,02 % du même condensat de monooléate de sorbitan et d'oxyde d'éthylène.

Après séchage pendant 4 heures, on contamine chaque plant par pulvérisation au moyen d'une suspension aqueuse de spores de Phytophthora infestans, responsable du mildiou de la tomate, à raison d'environ 1ml/plant (soit environ $2 \cdot 10^5$ spores par plant).

Après cette contamination, les plants de tomate sont mis en incubation pendant trois jours à 20 °C environ en atomospshère saturée d'humidité, puis pendant quatre jours à 17 °C environ sous 70 % à 80 % d'humidité relative.

Sept jours après la contamination, on compare les résultats obtenus dans le cas des plants traités par la matière active à tester à ceux obtenus dans le cas des plants utilisés comme témoins et on détermine la concentration minimale inhibitrice entraînant de 95 à 100 % d'inhibition du développement du champignon considéré (CNI 95-100).

Dans ces conditions, on observe que, pour les composés ou mélanges de composés décrits dans les exemples précédents, cette concentration a été respectivement la suivante :

| Composé ou mélange testé | CMI (95-100) Phytophthora infestans en mg/l |
|---|---|
| 1 | 62 |
| 2A | 4 |
| 2B | 4 |
| 2A + 2B | 4 |
| 3A + 3B | 8 |
| 4A + 4B | 31 |
| 5A + 5B | 2 |
| 6A + 6B | 62 |
| 7A + 7B | 2 |
| 7A | 2 |
| 7B | 4 |
| 9 | 4 |
| 10 | inférieure ou égale à 4 |
| 12A + 12B | 125 |
| 13A + 13B | 250 |
| 14 | 4 |
| 15A + 15B | 31 |
| 16 | 31 |
| 18 | 125 |
| 19A + 19B | 62 |
| 20 | 250 |
| 21 | 16 |
| 22 | inférieure ou égale à 250 |
| 23 | 8 |
| 24 | inférieure ou égale à 31 |
| 25A + 25B | 31 |
| 26 | 8 |
| 27 | 15 |
| 29A + 29B | 4 |
| 39A + 30B | 250 |
| 31A + 31B | 16 |
| 32A + 32B | 31 |
| 36A + 36B | 125 |
| 40A + 40B | inférieure ou égale à 125 |
| 41A + 41B | 16 |
| 42 | 500 |
| 44 | 250 |
| 48 | inférieure ou égale à 125 |

| Composé ou mélange testé | CMI (95-100) Phytophthora infestans en mg/l |
|---|---|
| 49 | inférieure ou égale à 4 |
| 51 | 8 |
| 52A + 52B | 125 |
| 53A + 53B | 31 |
| 55 | 250 |
| 56A + 56B | 1 |
| 57 | 8 |
| 58A + 58B | 16 |
| 59A + 59B | 31 |
| 60A + 60B | 4 |
| 61 | 1 |
| 63A + 63B | 4 |
| 64 | 8 |
| 65A + 65B | inférieure ou égale à 4 |
| 66 | 500 |
| 67 | inférieure ou égale à 500 |
| 68A + 68B | inférieure ou égale à 4 |

Dans cet essai on a observé des pourcentages d'inhibition du développement du champignon considéré compris entre 20 % et 90 % :

— à la dose de 500 mg/l pour les composés n° 8, 11, 28, 43, 47, 50 et pour les mélanges n° (35A + 35B), (46A + 46B), (62A + 62B) et (54A + 54B)

— à la dose de 15 mg/l pour les composés n° 17, (33A + 33B) (37A + 37B)

— à la dose de 8 mg/l pour le composé n° 39

— à la dose de 4 mg/l pour les composés n° 45.

Les mélanges cités dans cet exemple et le suivant sont ceux dont la composition pondérale a été donnée dans le tableau décrit plus haut.

Comparaison

Le cyano-2 benzimidazole, décrit dans les deux documents cités plus haut, et testé dans les mêmes conditions, a montré une CMI 95-100 de 2 000 mg/l, donc très supérieure à celle (62 mg/l) du diméthylsulfamoyl-1-cyano-2 benzimidazole (composé 1 décrit plus haut).

On observe donc que, d'après ce test que le remplacement de l'atome d'hydorgène situé sur l'azote en position 1 sur le cycle, par le radical diméthylsulfamoyle permet d'augmenter considérablement l'activité antifongique.

Exemple 8 : Test en serre sur mildiou du tabac

On opère comme à l'exemple précédent si ce n'est que les végétaux sont des plants de tabac (Nicotiana tabacum) de variété Samson et que ces plants sont contaminés par des spores de Peronospora tabacina, responsable du mildiou du tabac.

Dans ces conditions, on a observé que, pour les composés ou mélanges de composés décrits dans les exemples précédents, les concentrations minimales inhibitrices entraînant de 95 à 100 % d'inhibition du champignon considéré (CMI 95-100) sont respectivement les suivantes :

| Composés ou mélange testés | CMI (95-100) Peronospora tabaci en mg/l |
|---|---|
| 1 | 30 |
| 2A | 2 |
| 2B | 2 |
| 2A + 2B | 2 |
| 3A + 3B | 2 |
| 5A + 5B | 1 |
| 6A + 6B | supérieure ou égale à 62 |
| 7A + 7B | 2 |
| 7A | 4 |
| 7B | 4 |
| 9 | 2 |
| 10 | 62 |

| Composés ou mélange testés | CMI (95-100) Peronospora tabaci en mg/l |
|---|---|
| 11 | 62 |
| 13A + 13B | 125 |
| 14 | 1 |
| 15A + 15B | 4 |
| 18 | 62 |
| 19A + 19B | 15 |
| 21 | 16 |
| 23 | 8 |
| 24 | 31 |
| 25A + 25B | 1 |
| 26 | 31 |
| 27 | 125 |
| 29A + 29B | 2 |
| 30A + 30B | 125 |
| 36A + 36B | 500 |
| 39 | 62 |
| 40A + 40B | 16 |
| 45 | 500 |
| 48 | inférieure ou égale à 500 |
| 49 | 16 |
| 50 | inférieure ou égale à 125 |
| 51 | 32 |
| 52A + 52B | 500 |
| 55 | 62 |
| 56A + 56B | 1 |
| 58A + 58B | 16 |
| 59A + 59B | 500 |
| 60A + 60B | 2 |
| 61 | 8 |
| 62A + 62B | supérieure ou égale à 500 |
| 63A + 63B | 4 |
| 65A + 65B | 4 |
| 66 | 500 |
| 67 | inférieure ou égale à 500 |

Dans cet essai on a observé des pourcentages d'inhibition du développement du champignon considéré compris entre 20 % et 90 % :

à la dose de 500 mg/l pour les composés n° 38, 42, et 44 et pour les mélanges n° (31A + 31B), (41A + 41B) et (53A + 53B), 28,

à la dose de 15 mg/l pour les composés n° 16, 22, 57, 64 et pour les mélanges n° (4A + 4B), (12A + 12B), (35A + 35B), (37A + 37B) et (54A + 54B), à la dose de 8 mg/l pour le composé n° 21.

Comparaison

Le cyano-2 benzimidazole, décrit dans les documents cités plus haut, testé selon les mêmes conditions n'a montré aucune activité antifongique à la dose de 1 000 mg/l (% d'inhibition = 0 %).

Dans les mêmes conditions le diméthylsulfamoyl-1 cyano-2 benzimidazole (composé n° 1) présente une concentration minimale inhibitrice (CMI 95-100) égale à 30 mg/l.

Exemple n° 9 : Activité acaricide par contact-ingestion (feuillage traité par trempage ; Tetranychus urticae koch, femelles parthénogénétiques).

On prépare, une émulsion aqueuse de la matière active à tester ; par broyage dans un broyeur de Potter ; dans de l'eau contenant 0,02 % de Tween 80. L'émulsion aqueuse est ensuite amenée à la concentration désirée par dilution dans de l'eau contenant 0,01 % de Scurol 0.

Le Tween 80 est un condensat de monoléate de sorbitan et de 20 molécules d'oxyde d'éthylène.

Le Scurol 0 est un condensat d'octylphénol et de 10 moles d'oxyde d'éthylène.

On utilise des plants de haricot (phascolus vulgaris — variété Contender) au stade « feuilles cotylédonaires ». Chaque plant est traité par trempage des feuilles, pendant 10 secondes, dans l'émulsion aqueuse contenant la matière active à tester, à la concentration voulue, en utilisant deux plants pour chaque concentration. L'essai a été effectué pour des concentrations en matière active allant de 200 mg/l à 10 mg/l. Les plants de haricot sont maintenus en vie par immersion des racines et de la base de la tige dans de l'eau distillée.

Après séchage de la surface foliaire, la contamination s'effectue en déposant sur chaque feuille de haricot un fragment de feuille fortement contaminé provenant de l'élevage d'acariens. Ce fragment de feuille est retiré au bout de 24 heures.

Trois jours après la contamination, on dénombre, sous loupe binoculaire, le nombre d'acariens morts et le nombre d'acariens vivants.

On détermine ainsi, pour chaque concentration, le pourcentage de mortalité (moyenne de deux essais). A partir de ces pourcentages, on détermine la concentration ($Cl_{90}$) qui entraîne une mortalité de 90 % des acariens. Pour les composés ou mélanges de composés décrits précedemment, cette concentration est la suivante :

| | |
|---|---|
| 3A + 3B | 26 mg/l |
| 4A + 4B | 900 mg/l |
| 5A + 5B | 70 mg/l |
| 56A + 56B | 30 mg/l |
| 60A + 60B | 30 mg/l |
| 61 | 2 000 mg/l |
| 65A + 65B | supérieure à 2 000 mg/l |
| 70 | 10 mg/l |
| 71A + 71B | 30 mg/l |

Les exemples n° 7 et 8 décrits plus haut illustrent la bonne activité antifongique des composés selon l'invention. Dans un autre essai, effectué sur vigne au moyen des mélanges n° 2A + 2B et 7A + 7B, il a été observé que des traitements effectués au moyen de bouillies contenant de 15 à 60 g/hl de l'un ou l'autre de ces mélanges et appliquées jusqu'à limite de ruissellement sur les plants de vigne, assuraient une bonne protection contre mildiou de la vigne (Plasmopara viticola).

L'exemple n° 9 décrit plus haut illustre la bonne activité acaricide, par contact-ingestion de certains des composés selon l'invention. Dans un autre essai effectué sur feuilles de haricot contaminés par des œufs du même acarien que dans cet exemple n° 9, il a été observé que ces mêmes composés présentaient une bonne activité acaricide/ovicide par contact.

Les composés selon l'invention sont avantageusement utilisés comme antifongiques dans le domaine agricole. Ils présentent une action par contact et par systèmie et peuvent être employés à titre préventif et/ou à titre curatif pour la lutte contre divers champignons phytopathogènes tels que par exemple de nombreux phycomycètes et basidiomycètes. Comme indiqué plus haut, certains de ces composés peuvent être également utilisés avantageusement comme antiacariens notamment vis à vis des acariens phytophages.

Pour leur emploi dans la pratique, les composés selon l'invention ne sont généralement pas utilisés seuls. Le plus souvent ils sont utilisés dans des compositions qui comprennent en général, en plus de la matière active, un support (ou diluant) inerte et/ou un agent tensioactif compatibles avec la matière active.

Ces compositions font également partie de la présente invention. Elles contiennent habituellement de 0,001 à 95 % en poids de matière active. Leur teneur en agent tensioactif est en général comprise entre 0 % et 20 % en poids.

Par le terme « support », dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment par la plante traitée. Le support peut être solide (argiles, silicates naturels ou synthétiques, silice, craies, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques ; des sels d'acides lignosulfoniques des sels d'acides phénolsulfoniques ou naphtalènesulfoniques ; des polycondensats d'oxyde d'éthylène sur des alcools gras ou sur des acides gras ou sur des amines grasses ou sur des phénols substitués (notamment des alkylphénols ou des arylphénols ou le styrylphénol) ; des sels d'esters d'acides sulfosucciniques ; des dérivés de la taurine (notamment des alkyltaurates) ; des esters phosphoriques d'alcools ou de phénols polyoxyéthylés. La présence d'au moins un agent tensioactif est généralement indispensable surtout lorsque le support inerte n'est pas soluble dans l'eau, et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les émulsions, les suspensions

concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matières actives, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matières actives, et elles contiennent habituellement, en plus ou à la place du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici une composition de poudre mouillable à 50 % :

| | |
|---|---|
| — matière active (mélange 50/50 des composés 2A et 2B) | 50 % |
| — alcool gras éthoxylé (agent mouillant) | 2,5 % |
| — styrylphénol éthoxylé (agent dispersant) | 5 % |
| — craie (support inerte) | 42,5 % |

Un autre exemple de poudre mouillable a la composition suivante :

| | |
|---|---|
| — matière active (mélange 50/50 des composés 5A et 5B) | 90 % |
| — alcool gras éthoxylé (agent mouillant) | 4 % |
| — styrylphénol éthoxylé (agent dispersant) | 6 % |

Un autre exemple de poudre mouillable à 50 % a la composition suivante :

| | |
|---|---|
| — matière active (mélange 50/50 des composés 7A et 7B) | 50 % |
| — mélange de tensio-actifs anioniques et non ioniques (agent mouillant) | 2,5 % |
| — lignosulfonate de sodium neutre (agent dispersant) | 5 % |
| — argile kaolinique (support inerte) | 42,5 % |

Cette dernière composition a été utilisée dans le cas des traîtements sur vigne mentionnés plus haut.

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Comme cela a déjà été dit, les dispersions et émulsions aqueuses, par exemples des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huide-dans-l'eau et elles peuvent avoir une consistance épaisse comme celle d'une « mayonnaise ».

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Les composés de formule (II) peuvent encore être utilisés sous forme de poudres pour poudrage, on peut ainsi utiliser une composition comprenant 50 g de matière active et 950 g de talc ; on peut aussi utiliser une composition comprenant 20 g de matière active, 10 g de silice finement divisée et 970 g; on mélange et broie ces constituants et on applique le mélange par poudrage.

L'invention concerne de plus un procédé de traitement des végétaux contre les champignons phytopathogènes.

Ce procédé est caractérisé en ce qu'il consiste à appliquer sur ces végétaux une quantité efficace d'une composition contenant comme matière active un composé selon la formule (II). Par « quantité efficace » on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 5 g/hl à 100 g/hl correspondant sensiblement à des doses de matière active par hectare de 50 g/ha à 1000 g/ha environ donnent généralement de bons résultats.

L'invention concerne enfin un procédé de traîtement des végétaux contre les acariens phytophages selon lequel on applique sur ces végétaux une quantité efficace d'une composition contenant comme matière active un composé acaricide répondant à la formule (II). En pratique des doses allant de 10 à 100 g/hl environ donnent généralement de bons résultats.

**Revendications** (pour les Etats contractants : BE, CH, DE, FR, GB, IT, LI, LU, NL, SE)

1. Dérivé du cyano-2 benzimidazole caractérisé en ce qu'il répond à la formule générale :

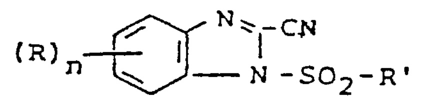

dans laquelle :

n représente un nombre entier égal à 0, 1, 2, ou 3,

R représente un atome d'halogène ou un radical alkyle inférieur, éventuellement substitué par un ou plusieurs atomes d'halogènes ; alkoxy inférieur éventuellement substitué par un ou plusieurs atomes d'halogènes ou par le radical phényle ; alkylthio inférieur éventuellement substitué par un ou plusieurs atomes d'halogènes ; alcényle inférieur ; alcynyle inférieur ; amino : nitro ; cyano ; cyanato ; thiocyanato ; isothiocyanato ; alkylsulfonyl inférieur ; sulfamoyle, alkylsulfinyl inférieur ; benzoyle ; alkoxycarbonyle comportant de 2 à 5 atomes de carbone ; étant entendu que, lorsque n est supérieur ou égal à 2, les substituants R peuvent être soit identiques soit différents ;

R′ représente un radical alkyle ou cycloalkyle inférieurs, éventuellement substitués par un ou plusieurs atomes d'halogènes ; ou un radical amino éventuellement substitué par un ou deux radicaux alkyles inférieurs, identiques ou différents, eux-mêmes éventuellement substitués par un radical alkoxy inférieur ; ou un atome d'azote substitué par deux radicaux formant avec cet atome d'azote un hétérocycle, comportant de 4 à 6 chaînons et de 1 à 3 hétéroatomes dans le cycle ; étant entendu que dans ce qui précède l'adjectif « inférieur », appliqué à un radical organique signifie que ce radical comporte au plus 6 atomes de carbone.

2. Composé selon la revendication 1 caractérisé en ce que

n et R ont la même signification que dans la revendication 1 ;

R′ représente le radical diméthylamino ou un radical alkyle comportant de 1 à 3 atomes de carbone, éventuellement substitué par un ou plusieurs halogènes ; ou un atome d'azote substitué par deux radicaux formant avec cet atome d'azote un hétérocycle comportant de 4 à 6 chaînons et comportant éventuellement comme deuxième hétéroatome dans le cycle l'atome d'oxygène.

3. Composition pesticide, à usage agricole, utilisable notamment pour la lutte contre les champignons phytopathogènes, caractérisée en ce qu'elle comprend comme matière active un composé selon la revendication 1.

4. Composition selon la revendication 3 utilisable pour la lutte contre les acariens phytophages caractérisée en ce qu'elle comprend comme matière active un composé répondant à la formule générale indiquée comme la revendication 1, dans laquelle n est égal à 1, 2 ou 3 et l'un au moins des substituants R représente le radical trifluorométhyle.

5. Composition selon l'une des revendications 3 et 4 caractérisée en ce que, en plus de la matière active, elle comprend un support inerte et/ou un agent tensioactif utilisables en agriculture.

6. Composition selon la revendication 5 caractérisée en ce qu'elle comprend de 0,001 % à 95 % en poids de matière active et de 0 à 20 % en poids d'agent tensioactif.

7. Procédé de préparation d'un composé selon la revendication 1) caractérisé en ce qu'il consiste à faire réagir un cyano-2 benzimidazole de formule :

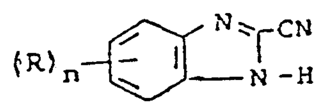

dans laquelle R et n ont la même signification que dans la revendication 1), ou un sel de métal alcalin ou d'ammonium de ce cyano-2 benzimidazole, sur un halogénure de formule :

$$X—SO_2R'$$

dans laquelle R′ a la même signification que dans la revendication 1) et X représente un atome d'halogène.

8. Procédé selon la revendication 7) caractérisé en ce que la réaction entre le cyano-2 benzimidazole et l'halogénure s'effectue dans un solvant aprotique polaire, en présence d'un accepteur d'acide.

9. Procédé selon la revendication 7) caractérisé en ce que la réaction entre le sel de métal alcalin ou

d'ammonium du cyano-2 benzimidazole et l'halogénure s'effectue dans un solvant aprotique polaire.

10. Procédé de traitement des végétaux contre les champignons phytopathogènes caractérisé en ce qu'il consiste à appliquer sur ces végétaux une quantité efficace d'un composé selon la revendication 1).

11. Procédé de traîtement des végétaux contre les acariens phytophages, caractérisée en ce qu'il consiste à appliquer sur ces végétaux une quantité efficace d'un composé selon la revendication 1 dans laquelle n est égal à 1, 2 ou 3 et l'un au moins des substituants R représente le radical trifluorométhyle.

**Revendications** (Pour l'Etat contractant AT)

1. Composition pesticide à usage agricole, utilisable notamment pour la lutte contre les champignons phytopathogènes, caractérisée en ce qu'elle comprend, comme matière active, un composé répondant à la formule (II)

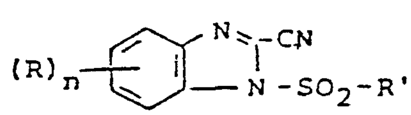

(II)

dans laquelle :

n représente un nombre entier égal à 0, 1, 2, ou 3,

R représente un atome d'halogène ou un radical alkyle inférieur, éventuellement substitué par un ou plusieurs atomes d'halogènes ; alkoxy inférieur éventuellement substitué par un ou plusieurs atomes d'halogènes ou par le radical phényle ; alkylthio inférieur éventuellement substitué par un ou plusieurs atomes d'halogènes ; alcényle inférieur ; alcynyle inférieur ; amino ; nitro ; cyano ; cyanato ; thiocyanato ; isothiocyanato ; alkylsulfonyle inférieur ; sulfamoyle ; alkylsulfinyle inférieur ; benzoyle ; alkoxycarbonyle comportant de 2 à 5 atomes de carbone ; étant entendu que, lorsque n est supérieur ou égal à 2, les substituants R peuvent être soit identiques soit différents ;

R' représente un radical alkyle ou cycloalkyle inférieurs, éventuellement substitués par un ou plusieurs atomes d'halogènes ; ou un radical amino éventuellement substitués par un ou deux radicaux alkyles inférieurs, identiques ou différents, eux-mêmes éventuellement substitués par un radical alkoxy inférieur ou un atome d'azote substitué par deux radicaux formant avec cet atome d'azote un hétérocycle, comportant de 4 à 6 chaînons et de 1 à 3 hétéroatomes dans le cycle ; étant entendu que dans ce qui précède l'adjectif « inférieur », appliqué à un radical organique signifie que ce radical comporte au plus 6 atomes de carbone.

2. Composition selon la revendication 1 caractérisée en ce qu'elle comprend, comme matière active, un composé répondant à la formule (II) dans laquelle :

n et R ont la même signification que dans la revendication 1 ;

R' représente le radical diméthylamino ou un radical alkyle comportant de 1 à 3 atomes de carbone, éventuellement subsitué par un ou plusieurs halogènes ; ou un atome d'azote substitué par deux radicaux formant avec cet atome d'azote un hétérocycle comportant de 4 à 6 chaînons et comportant éventuellement, comme deuxième hétéroatome dans le cycle, l'atome d'oxygène.

3. Composition selon la revendication 1, utilisable pour la lutte contre les acariens phytophages, caractérisée en ce qu'elle comprend, comme matière active, un composé répondant à la formule (II) dans laquelle n est égal à 1, 2 ou 3 et l'un au moins des substituants, R représente le radical trifluorométhyle.

4. Composition selon l'une des revendications 2 et 3 caractérisée en ce que, en plus de la matière active, elle comprend un support inerte et/ou un agent tensioactif utilisables en agriculture.

5. Composition selon la revendication 4 caractérisée en ce qu'elle comprend de 0,001 % à 95 % en poids de matière active et de 0 à 20 % en poids d'agent tensioactif.

6. Procédé de préparation d'un composé répondant à la formule (II)

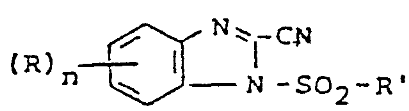

(II)

dans laquelle n, R et R' ont la même signification que dans la revendication 1 caractérisé en ce qu'il consiste à faire réagir un cyano-2 benzimidazole de formule :

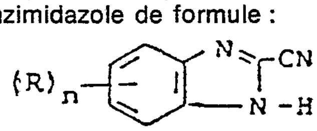

dans laquelle R et n ont la même signification que précédemment, ou un sel de métal alcalin ou d'ammonium de ce cyano-2 benzimidazole, sur un halogénure de formule :

$X—SO_2R'$

dans laquelle R' a la même signification que précédemment et X représente un atome d'halogène.

7. Procédé selon la revendication 6 caractérisé en ce que la réaction entre le cyano-2 benzimidazole et l'halogénure s'effectue dans un solvant aprotique polaire, en présence d'un accepteur d'acide.

8. Procédé selon la revendication 6 caractérisé en ce que la réaction entre le sel de métal alcalin ou d'ammonium du cyano-2 benzimidazole et l'halogénure s'effectue dans un solvant aprotique polaire.

9. Procédé de traitement des végétaux contre les champignons phytopathogènes caractérisé en ce qu'il consiste à appliquer sur ces végétaux une quantité efficace d'un composé répondant à la formule (II)

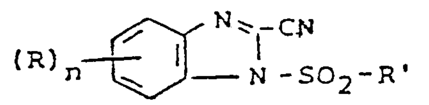 (II)

dans laquelle n, R et R' ont la même signification que dans la revendication 1.

10. Procédé de traitement des végétaux contre les acariens phytophages, caractérisé en ce qu'il consiste à appliquer sur ces végétaux une quantité efficace d'un composé répondant à la formule (II) dans laquelle n est égal à 1, 2, ou 3 et l'un au moins des substituants, R représente le radical trifluorométhyle.

**Claims** (for the Contracting States : BE, CH, DE, FR, GB, IT, LI, LU, NL, SE)

1. 2-cyanobenzimidazole derivative characterized in that it corresponds to the general formula :

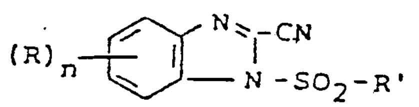

in which :

n represents an integer equal to 0, 1, 2 or 3 ;

R represents a halogen atom or a lower alkyl radical optionally substituted by one or more halogen atoms ; a lower alkoxy radical optionally substituted by one or more halogen atoms or by the phenyl radical ; a lower alkylthio radical optionally substituted by one or more halogen atoms ; a lower alkenyl radical ; a lower alkynyl radical ; an amino radical ; a nitro radical ; a cyano radical ; a cyanato radical ; a thiocyanato radical ; an isothiocyanato radical ; a lower alkylsulphonyl radical ; a sulphamoyl radical ; a lower alkylsulphinyl radical ; a benzoyl radical ; or an alkoxycarbonyl radical containing from 2 to 5 carbon atoms, it being understood that if n is greater than or equal to 2, the substituents R can be either identical or differents ; and

R' represents a lower alkyl or cycloalkyl radical optionally substituted by one or more halogen atoms ; an amino radical optionally substituted by one or two identical or different lower alkyl radicals which are themselves optionally substituted by a lower alkoxy radical ; or a nitrogen atom substituted by two radicals forming a heterocyclic ring with this nitrogen atom, the heterocyclic ring containing from 4 to 6 ring members and from 1 to 3 hetero-atoms in the ring, it being understood that, in the above, the adjective « lower » applied to an organic radical means that this radical contains at most 6 carbon atoms.

2. Compound according to claim one, characterized in that

n and R have the same meaning as in claim 1, and

R' represents the dimethylamino radical, an alkyl radical containing from 1 to 3 carbon atoms, optionally substituted by one or more halogens, or a nitrogen atom substituted by two radicals forming a heterocyclic ring with this nitrogen atom, the heterocyclic ring containing from 4 to 6 ring members and optionally containing the oxygen atom as a second hetero-atom in the ring.

3. Pesticidal composition for agricultural use, usable in particular for combating phytopathogenic fungi, characterized in that it comprises as active material a compound corresponding to claim 1.

4. Composition according to claim 3 usable for combating plant-eating mites, characterized in that it comprises as active ingredient a compound corresponding to the general formula indicated in claim 1, in which n is equal to 1, 2 or 3 and at least one of the substituents R represents the trifluoromethyl radical.

5. Composition according to one of claims 4 and 5, characterized in that it comprises, in addition to the active ingredient, an inert carrier and/or a surface-active agent which can be used in agriculture.

6. Composition according to claim 5, characterized in that it comprises from 0.001 % to 95 % by weight of active ingredient and from 0 to 20 % by weight of surface-active agent.

7. Process for the preparation of a compound according to claim 1, characterized in that it consists in reacting 2-cyanobenzimidazole of the formula :

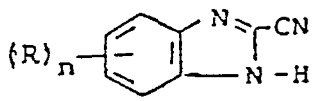

in which R and n have the same meaning as in claim 1, or an alkali metal salt or ammonium salt of this 2-cyanobenzimidazole, with a halide of the formula :

$$X—SO_2R'$$

in which R' has the same meaning as in claim 1 and X represents a halogen atom.

8. Process according to claim 7, characterized in that the reaction of the 2-cyanobenzimidazole with the halide is carried out in a polar aprotic solvent, in the presence of an acid acceptor.

9. Process according to claim 7, characterized in that the reaction of the alkali metal salt or ammonium salt of the 2-cyanobenzimidazole with the halide is carried out in a polar aprotic solvent.

10. Process for the treatment of plants to combat phytopathogenic fungi, characterized in that it consists in applying to these plants an effective amount of a compound according to claim 1.

11. Process for the treatment of plants to combat plant-eating mites, characterized in that it consists in applying to these plants, an effective amount of a compound according to claim 1 in which n is equal to 1, 2 or 3 and at least one of the substituents R represents the trifluoromethyl radical.

**Claims** (for the Contracting State AT)

1. Pesticidal composition for agricultural use, usable in particular for combating phytopathogenic fungi, characterized in that it comprises as actives ingredient a compound corresponding to the formula (II) :

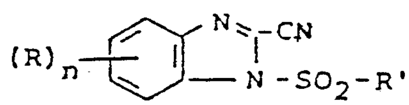

in which :

n represents an integer equal to 0, 1, 2 or 3 ;

R represents a halogen atom or a lower alkyl radical optionally substituted by one or more halogen atoms ; a lower alkoxy radical optionally substituted by one or more halogen atoms or by the phenyl radical ; a lower alkylthio radical optionally substituted by one or more halogen atoms ; a lower alkenyl radical ; a lower alkynyl radical ; an amino radical ; a nitro radical ; a cyano radical ; a cyanato radical ; a thiocyanato radical ; an isothiocyanato radical ; a lower alkylsulphonyl radical ; a sulphamoyl radical ; a lower alkylsulphinyl radical ; a benzoyl radical ; or an alkoxycarbonyl radical containing from 2 to 5 carbon atoms, it being understood that if n is greater than or equal to 2, the substituents R can be either identical or differents ; and

R' represents a lower alkyl or cycloalkyl radical optionally substituted by one or more halogen atoms ; an amino radical optionally substituted by one or two identical or different lower alkyl radicals which are themselves optionally substituted by a lower alkoxy radical ; or a nitrogen atom substituted by two radicals forming a heterocyclic ring with this nitrogen atom, the heterocyclic ring containing from 4 to 6 ring members and from 1 to 3 hetero-atoms in the ring, it being understood that, in the above, the adjective « lower » applied to an organic radical means that this radical contains at most 6 carbon atoms.

2. Composition according to claim 1, characterised in that it comprises as active ingredient a compound corresponding to the formula (II) in which :

n and R have the same meaning as in claim 1, and

R' represents the dimethylamino radical, an alkyl radical containing from 1 to 3 carbon atoms and optionally substituted by one or more halogens, or a nitrogen atom substituted by two radicals forming a heterocyclic ring with this nitrogen atom, the heterocyclic ring containing from 4 to 6 ring numbers and optionally containing the oxygen atom as a second hetero-atom in the ring.

3. Composition according to claim 1, usable for combating plant-eating mites, characterized in that it comprises, as the active ingredient, a compound corresponding to the formula (II) in which n is equal to 1, 2 or 3 and at least one of the substituents R represents the trifluoromethyl radical.

4. Composition according to one of claims 2 and 3, characterized in that it comprises, in addition to the active ingredient, an inert carriet and/or a surface-active agent which can be used in agriculture.

5. Composition according to claim 4, characterized in that it comprises from 0.001 % to 95 % by weight of active ingredient and from 0 to 20 % by weight of surface-active agent.

6. Process for the preparation of a compound corresponding to the formula (II) :

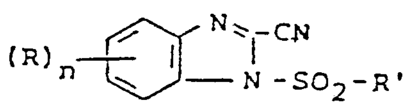

in which n, R and R′ have the same meaning as in claim 1, characterized in that it consists in reacting a 2-cyanobenzimidazole of the formula :

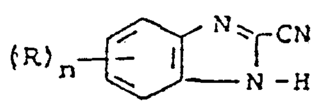

in which R and n have the same meaning as before, or an alkali metal salt or ammonium salt of this 2-cyanobenzimidazole, with a halide of the formula :

$$X—SO_2R'$$

in which R′ has the same meaning as before and X represents a halogen atom.

7. Process according to claim 6, characterized in that the reaction of the 2-cyanobenzimidazole with the halide is carried out in a polar aprotic solvent, in the presence of an acid acceptor.

8. Process according to claim 6, characterized in that the reaction of the alkali metal salt or ammonium salt of the 2-cyanobenzimidazole with the halide is carried out in a polar aprotic solvent.

9. Process for the treatment of plants to combat phytopathogenic fungi, characterized in that it consists in applying to these plants an effective amount of a compound according to the formula (II) :

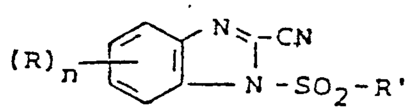

in which n, R and R′ have the same meaning as in claim 1.

10. Process for the treatment of plants to combat plant-eating mites, characterized in that it consists in applying to these plants an effective amount of a compound according to the formula (II) in which n is equal to 1, 2 or 3 and at least one of the substituents R represents the trifluoromethyl radical.

**Patentansprüche** (für die Vertragsstaaten : BE, CH, DE, FR, GB, IT, LI, LU, NL, SE)

1. 2-cyanobenzimidazol-Derivate gekennzeichnet durch die allgemeine Formel

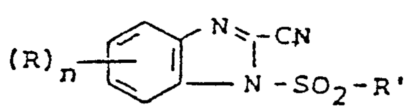

in der bedeuten :

n 0, 1, 2 oder 3,

R Halogen, ggf ein- oder mehrfach halogensubstituiertes niederes Alkyl, ggf ein- oder mehrfach halogensubstituiertes oder phenylsubstituiertes niederes Alkoxy, ggf ein- oder mehrfach halogensubstituiertes niederes Alkylthio, niederes Alkenyl, niederes Alkinyl, Amino, Nitro, Cyano, Cyanato, Thiocyanato, Isothiocyanato, niederes Alkylsulfonyl, Sulfamoyl, niederes Alkylsulfinyl, Benzoyl oder $C_2$- bis $C_5$-Alkoxycarbonyl, mit der Maßgabe, daß die Substituenten R gleich oder verschieden sind, wenn $n \geq 2$ ist,

R′ niederes Alkyl oder niederes Cycloalkyl, die ggf ein- oder mehrfach halogensubstituiert sind, ggf mit einer oder zwei gleichen oder verschiedenen niederen Alkylgruppen substituiertes Amino, die ihrerseits ggf mit einer niederen Alkoxygruppe substituiert sind, oder ein mit zwei Gruppen substituiertes Stickstoffatom, die mit ihm einen 4- bis 6-gliedrigen Heterocyclus mit 1 bis 3 Heteroatomen bilden, wobei « nieder » einen organischen Rest mit höchstens 6 Kohlenstoffatomen bedeutet.

2. Derivate der Formel nach Anspruch 1,

in der bedeuten :

n und R dasselbe wie in Anspruch 1 und

R′ Dimethylamino, ggf ein- oder mehrfach halogensubstituiertes $C_1$- bis $C_3$-Alkyl oder ein mit zwei Gruppen substituiertes Stickstoffatom, die mit ihm einen 4- bis 6-gliedrigen Heterocyclus bilden, der ggf ein Sauerstoffatom als zweites Heteroatom im Ring enthält.

3. Pesticide Zusammensetzung zur Verwendung in der Landwirtschaft, insbesondere zur Bekämpfung phytophager Pilze, gekennzeichnet durch mindestens ein Derivat nach Anspruch 1 als Wirkstoff.

4. Zusammensetzung nach Anspruch 3 zur Bekämpfung phytophager Milben, gekennzeichnet durch

ein Derivat der allgemeinen Formel nach Anspruch 1, in der n die Zahlen 1, 2 oder 3 bedeutet und mindestens einer der Substituenten R Trifluormethyl ist, als Wirkstoff.

5. Zusammensetzung nach Anspruch 3 oder 4, dadurch gekennzeichnet, daß sie neben dem Wirkstoff einen inerten Träger und/oder ein grenzflächenaktives Mittel enthält, die zur Verwendung in der Landwirtschaft geeignet sind.

6. Zusammensetzung nach Anspruch 5, dadurch gekennzeichnet, daß sie 0,001 bis 95 Gew.-% Wirkstoff und 0 bis 20 Gew.-% grenzflächenaktives Mittel enthält.

7. Verfahren zur Herstellung der Derivate gemäß Anspruch 1 gekennzeichnet durch Umsetzung eines 2-Cyanobenzimidazols der Formel

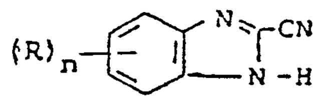

mit R und n wie in Anspruch 1 oder eines Alkalimetall- oder Ammoniumsalzes dieses 2-Cyanobenzimidazols mit einem Halogenid der Formel

$$X—SO_2R'$$

mit R' wie in Anspruch 1 und X = Halogen .

8. Verfahren nach Anspruch 7, dadurch gekennzeichnet, daß die Umsetzung des 2-Cyanobenzimidazols mit dem Halogenid in einem polaren aprotischen Lösungsmittel in Gegenwart eines Säureakkzeptors durchgeführt wird.

9. Verfahren nach Anspruch 7, dadurch gekennzeichnet, daß die Umsetzung des Alkalimetall- oder Ammoniumsalzes des 2-Cyanobenzimidazols mit dem Halogenid in einem polaren aprotischen Lösungsmittel durchgeführt wird.

10. Verfahren zur Behandlung von Pflanzen gegen phytopathogene Pilze, gekennzeichnet durch Anwendung einer wirksamen Menge eines Derivats des 2-Cyanobenzimidazols nach Anspruch 1 auf die Pflanzen.

11. Verfahren zur Behandlung von Pflanzen gegen phytophage Milben, gekennzeichnet durch Anwendung einer wirksamen Menge eines Derivats des 2-Cyanobenzimidazols nach Anspruch 1, in dessen Formel n 1, 2 oder 3 bedeutet und mindestens einer der Substituenten R Trifluormethyl ist, auf die Pflanzen.

**Patentansprüche** (für den Vertragsstaat AT)

1. Pesticide Zusammensetzung zur Verwendung in der Landwirtschaft, insbesondere zur Bekämpfung phytophager Pilze, gekennzeichnet durch eine Verbindung der Formel (II) als Wirkstoff

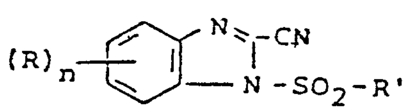

in der bedeuten :

n 0, 1, 2 oder 3,

R Halogen, ggf ein- oder mehrfach halogensubstituiertes niederes Alkyl, ggf ein- oder mehrfach halogensubstituiertes oder phenylsubstituiertes niederes Alkoxy, ggf ein- oder mehrfach halogensubstituiertes niederes Alkylthio, niederes Alkenyl, niederes Alkinyl, Amino, Nitro, Cyano, Cyanato, Thiocyanato, Isothiocyanato, niederes Alkylsulfonyl, Sulfamoyl, niederes Alkylsulfinyl, Benzoyl oder $C_2$- bis $C_5$-Alkoxycarbonyl, mit der Maßgabe, daß die Substituenten R gleich oder verschieden sind, wenn $n \geqslant 2$ ist,

R' niederes Alkyl oder niederes Cycloalkyl, die ggf ein- oder mehrfach halogensubstituiert sind, ggf mit einer oder zwei gleichen oder verschiedenen niederen Alkylgruppen substituiertes Amino, die ihrerseits ggf mit einer niederen Alkoxygruppe substituiert sind, oder ein mit zwei Gruppen substituiertes Stickstoffatom, die mit ihm einen 4- bis 6-gliedrigen Heterocyclus mit 1 bis 3 Heteroatomen bilden, wobei « nieder » einen organischen Rest mit höchstens 6 Kohlenstoffatomen bedeutet.

2. Zusammensetzung nach Anspruch 1, gekennzeichnet durch eine Verbindung der Formel (II) als Wirkstoff,

in der bedeuten :

n und R dasselbe wie in Anspruch 1 und

R' Dimethylamino, ggf ein- oder mehrfach halogensubstituiertes $C_1$- bis $C_3$-Alkyl oder ein mit zwei Gruppen substituiertes Stickstoffatom, die mit ihm einen 4- bis 6-gliedrigen Heterocyclus bilden, der ggf ein Sauerstoffatom als zweites Heteroatom im Ring enthält.

3. Zusammensetzung nach Anspruch 1 zur Bekämpfung phytophager Milben, gekennzeichnet durch eine Verbindung der Formel (II) als Wirkstoff, in der n die Zahlen 1, 2 oder 3 bedeutet und mindestens einer der Substituenten R Trifluormethyl ist.

4. Zusammensetzung nach Anspruch 2 oder 3, dadurch gekennzeichnet, daß sie neben dem Wirkstoff einen inerten Träger und/oder ein grenzflächenaktives Mittel enthält, die zur Verwendung in der Landwirtschaft geeignet sind.

5. Zusammensetzung nach Anspruch 4, dadurch gekennzeichnet, daß sie 0,001 bis 95 Gew.-% Wirkstoff und 0 bis 20 Gew.-% grenzflächenaktives Mittel enthält.

6. Verfahren zur Herstellung einer Verbindung der Formel (II)

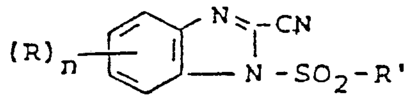

mit n, R und R' wie in Anspruch 1 gekennzeichnet durch Umsetzung eines 2-Cyanobenzimidazols der Formel

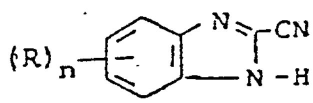

mit R und n wie in Anspruch 1 oder eines Alkalimetall- oder Ammoniumsalzes dieses 2-Cyanobenzimidazols mit einem Halogenid der Formel

$$X—SO_2R'$$

mit R' wie in Anspruch 1 und X = Halogen.

7. Verfahren nach Anspruch 6, dadurch gekennzeichnet, daß die Umsetzung des 2-Cyanobenzimidazols mit dem Halogenid in einem polaren aprotischen Lösungsmittel in Gegenwart eines Säureakkzeptors durchgeführt wird.

8. Verfahren nach Anspruch 6, dadurch gekennzeichnet, daß die Umsetzung des Alkalimetall- oder Ammoniumsalzes des 2-Cyanobenzimidazols mit dem Halogenid in einem polaren aprotischen Lösungsmittel durchgeführt wird.

9. Verfahren zur Behandlung von Pflanzen gegen phytopathogene Pilze, gekennzeichnet durch die Anwendung einer wirksamen Menge einer Verbindung der Formel (II) auf die Pflanzen

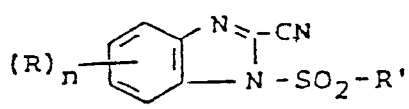

mit n, R und R' wie in Anspruch 1.

10. Verfahren zur Behandlung von Pflanzen gegen phytophage Milben, gekennzeichnet durch die Anwendung einer wirksamen Menge einer Verbindung der Formel (II), in der n 1, 2 oder 3 bedeutet und mindestens einer der Substituenten R Trifluormethyl ist, auf die Pflanzen.